(19) Europäisches Patentamt
European Patent Office
Office européen des brevets

(11) Publication number: **0 236 822**
**B1**

(12) **EUROPEAN PATENT SPECIFICATION**

(45) Date of publication of patent specification: **27.12.90**

(51) Int. Cl.[5]: **A 61 K 31/135,** A 61 K 47/00

(21) Application number: **87102448.5**

(22) Date of filing: **20.02.87**

# (54) Stable injectable pharmaceutical formulation for 1,4-dihydroxy-5,8-bis((2-(hydroxyethylamino)-ethyl)amino)anthraquinone, dihydrochloride.

(30) Priority: **07.03.86 US 837243**

(43) Date of publication of application:
**16.09.87 Bulletin 87/38**

(45) Publication of the grant of the patent:
**27.12.90 Bulletin 90/52**

(84) Designated Contracting States:
**AT BE CH DE ES FR GB GR IT LI NL SE**

(56) References cited:
**EP-A-0 003 150 US-A-3 230 143**
**EP-A-0 154 117 US-A-4 328 213**

**CHEMICAL ABSTRACTS, vol. 98, no. 6, February 1983, page 355, abstract no. 40504x, Columbus, Ohio, US; C. LIU et al.: "Studies on stability of catecholamine injections and improvement of formulation",**

**CHEMICAL ABSTRACTS, vol. 102, 1985, page 392, abstract no. 119532q, Columbus, Ohio, US; V. PANDOLFO et al.: "Stability of apomorphine parenteral formulations",**

(73) Proprietor: **AMERICAN CYANAMID COMPANY**
**1937 West Main Street P.O. Box 60**
**Stamford Connecticut 06904-0060 (US)**

(72) Inventor: **Niphadkar, Pradeep V.**
**201 Randolph Avenue**
**Clifton New Jersey 07011 (US)**
Inventor: **Desai, Narendra R.**
**Hollcroft Apts. 11H Clapboard Ridge Road**
**Danbury Connecticut 06810 (US)**
Inventor: **Jones, Paul N.**
**792 Brookridge Drive**
**Valley Cottage New York 10989 (US)**
Inventor: **Mooney, Kieran G.**
**30 Clinton Avenue**
**Warwick New York 10990 (US)**

(74) Representative: **Wächtershäuser, Günter, Dr.**
**Tal 29**
**D-8000 München 2 (DE)**

(56) References cited:
**CHEMICAL ABSTRACTS, vol. 90, 1979, page 388, abstract no. 210008t, Columbus, Ohio, US; M. STRUHAR et al.: "Studies on the stability and stabilization of aqueous solutions of emetine dichloride",**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 236 822 B1**

## Description

The compound 1,4-dihydroxy-5,8-bis[2-(2-hydroxyethylamino)ethylamino]anthraquinone, dihydrochloride, having the formula:

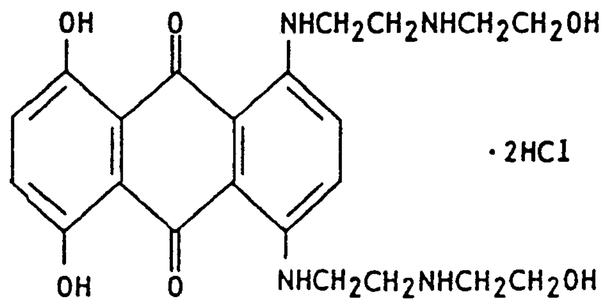

is described and claimed in U.S. Patent No. 4,197,249 and is sold under the registered trademark Novantrone® in Canada and Europe and is currently under consideration by the United States Food and Drug Administration for approval as an anti-cancer agent.

This compound is known to undergo oxidative degradation in aqueous solution. This degradation occurs much more rapidly in the presence of metal ions such as cuprous, cupric, ferrous and ferric, even when present in minute quantities (e.g. less than 10 ppm).

Since this compound exhibits optimum pharmacological activity in humans when administered parenterally (intramuscularly, intravenously) as opposed to oral administration, it is extremely important that solutions of this compound remain stable for prolonged period under normal storage conditions.

It has now been discovered that the inclusion of a combination of critical factors in an aqueous formulation of 1,4-dihydroxy-5,8-bis[2-(hydroxyethylamino)ethylamino]anthraquinone, dihydrochloride provides a formulation which is extremely stable under normal storage conditions.

These factors are 1) the inclusion of a suitable antioxidant, 2) specific pH range, and 3) the inclusion of a suitable metal ion chelator.

With regard to antioxidants the most effective proved to be sodium metabisulfite.

The most effective pH range was 2.0—3.5 with 3.0 being optimum.

The most effective metal ion chelator was a combination of disodium EDTA and glycine.

Since the desired injectable formulation of 1,4-dihydroxy-5,8-bis[2-(2-hydroxyethylamino)ethylamino]anthraquinone, dihydrochloride contains 1 to 5 mg of this compound per ml of formulation, this new stable formula would have a pH of 2.0 to 3.5, with 3.0 being optimal, a sodium metabilsulfite concentration of 0.01 to 0.10%, with 0.05% being optimal, disodium EDTA at a concentration of 0.01 to 0.11% with 0.10% being optimal, and glycine concentration of 0.05 to 0.2% iwth 0.1% being optimal.

In order to prove the enhanced stability of this new formulation, formulae of the following composition were prepared and stability studies conducted.

Formula I

| | |
|---|---|
| 1,4-Dihydroxy-5,8-bis[2-(2-hydroxyethylamino)ethylamino]anthraquinone, dihydrochloride | 2 mg/ml |
| Water for Injection U.S.P. | 100% |
| Headspace | Air |

Formula II

| | |
|---|---|
| 1,4-Dihydroxy-5,8-bis[2-(2-hydroxyethylamino)ethylamino]anthraquinone, dihydrochloride | 2 mg/ml |
| Cupric ions | 7 ppm |
| Water for Injection U.S.P. | 100% |
| Headspace | Air |
| pH | 5.50 |

Formula III

| | |
|---|---|
| 1,4-Dihydroxy-5,8-bis[2-(2-hydroxyethylamino)ethylamino]anthraquinone, dihydrochloride | 2 mg/ml |
| Sodium metabilsulfite | 0.01% |
| Sodium acetate | 0.005% |
| Acetic acid, glacial | 0.046% |
| Sodium chloride | 0.80% |
| Water for Injection U.S.P. | 100% |
| pH | 3.5 |
| Headspace | Nitrogen |

Formula IV

| | |
|---|---|
| 1,4-Dihydroxy-5,8-bis[2-(2-hydroxyethylamino)ethylamino]anthraquinone, dihydrochloride | 2 mg/ml |
| Sodium metabilsulfite | 0.05% |
| Disodium EDTA | 0.10% |
| Glycine | 0.10% |
| Sodium chloride (Isotonic Agent) | 0.786% |
| Water for Injection U.S.P. | 100% |
| pH | 3.0 |
| Headspace | as indicated* |

*One portion filled under nitrogen, two portions filled under air.

The results of these stability studies are given below:

| Formula | Vial Size | Headspace | Temp. °C | % of Initial Potency | | | | | |
|---|---|---|---|---|---|---|---|---|---|
| | | | | 1 Day | 2 Weeks | 3 Weeks | 4 Weeks | 6 Weeks | 8 Weeks |
| I | 10 ml | Air | 56 | | 88 | 84.5 | | 72 | |
| II | 10 ml | Air | 56 | 13 | | | | | |
| III | 10 ml | Nitrogen | 56 | | | | 90 | | 85 |
| III | 2 ml | Nitrogen | 42 | | | | 96 | | 93 |
| III | 2 ml | Nitrogen | 56 | | | | 85 | | 72 |
| IV | 10 ml | Nitrogen | 56 | | | | 99 | | |
| IV | 2 ml | Nitrogen | 42 | | | | 98.6 | | 97.9 |
| IV | 10 ml | Air | 56 | | | | 98 | | 97.6 |
| IV | 10 ml | Air | 42 | | | | 98 | | 97.5 |
| V | 10 ml | Nitrogen | 56 | | | | 99 | | 98.4 |

The above results show the superiority of the new formulation of this invention over the previous formulation and an aqueous solution of this drug.

Like most other anticancer drugs, the dose of Novantrone® is based upon the patient's body surface area and disease state. It is highly advantageous from cost and marketing point of view to have Novantrone® product available in small size containers like 2 ml vials. However, because of relatively higher headspace to volume ratio of smaller vials as against the larger vials, the stability of this product becomes critical in small 2 ml vials. It is important to note that the new formulation was found to be superior to the previous formulation in both small as well as relatively larger volume vials.

To further illustrate the stability of this invention a composition was prepared as follows.

Formula V

| | |
|---|---|
| 1,4-Dihydroxy-5,8-bis[2-(2-hydroxyethylamino)ethylamino]anthraquinone, dihydrochloride | 2 mg/ml |
| Disodium EDTA | 0.10% |
| Sodium metabilsulfite | 0.04% |
| Glycine | 0.10% |
| Sodium chloride (Isotonic Agent) | 0.60% |
| Water for Injection U.S.P. | 100% |
| pH | 3.0 |

This formula (V) and a composition of formula (I) each had cupric ions added to a concentration of 6 ppm and were then placed in a stability study at 56°C for 4 weeks. The results at the end of this time showed that formula (I) lost 98% of its potency in 2 days while formula (V) retained 98.6% potency after 4 weeks.

**Claims for the Contracting States: BE CH DE FR GB IT LI NL SE**

1. A pharmaceutical formulation comprising from 1 to 5 mg per ml of formulation of 1,4-dihydroxy-5,8-bis[2-(2-hydroxyethylamino)ethylamino]anthraquinone, dihydrochloride in an isotonic solution at a pH of from 2.0 to 3.5, containing sodium metabisulfite at a concentration of from 0.01 to 0.10%, disodium EDTA at a concentration of from 0.01 to 0.11% and glycine at a concentration of from 0.05 to 0.20%.

2. A formulation according to Claim 1 where the concentration of 1,4-dihydroxy-5,8-bis[2-(2-hydroxyethylamino)ethylamino]anthraquinone, dihydrochloride is 2 mg/ml and the concentrations of sodium metabisulfite, disodium EDTA and glycine are 0.05%, 0.10% and 0.10% respectively.

**Claims for the Contracting States: AT ES GR**

1. A method for preparing a pharmaceutical formulation which comprises mixing from 1 to 5 mg per ml of formulation of 1,4-dihydroxy-5,8-bis[2-(2-hydroxyethylamino)ethylamino]anthraquinone, dihydrochloride in an isotonic solution at a pH of from 2.0 to 3.5, containing sodium metabisulfite at a concentration of from 0.01 to 0.10%, disodium EDTA at a concentration of from 0.01 to 0.11% and glycine at a concentration of from 0.05 to 0.20%.

2. A method according to Claim 1, which comprises providing a formulation where the concentration of 1,4-dihydroxy-5,8-bis[2-(2-hydroxyethylamino)ethylamino]anthraquinone, dihydrochloride is 2 mg/ml and the concentrations of sodium metabisulfite, disodium EDTA and glycine are 0.05%, 0.10% and 0.10% respectively.

**Patentansprüche für die Vertragsstaaten: BE CH DE FR GB IT LI NL SE**

1. Pharmazeutische Formulierung umfassend von 1 bis 5 mg/ml Formulierung an 1,4-Dihydroxy-5,8-bis[2-(2-hydroxyethylamino)ethylamino]anthrachinondihydrochlorid in einer isotonischen Lösung bei einem pH von 2,0 bis 3,5, enthaltend Natriummetabilsulfid mit einer Konzentration von 0,01 bis 0,10%, Dinatrium EDTA mit einer Konzentration von 0,01 bis 0,11% und Glycin mit einer Konzentration von 0,05 bis 0,20%.

2. Formulierung gemäß Anspruch 1, wobei die Konzentration an 1,4-Dihydroxy-5,8-bis[2-(2-hydroxyethylamino)ethylamino]anthrachinondihydrochlorid 2 mg/ml beträgt und die Konzentrationen von Natriummetabisulfid, Dinatrium EDTA und Glycin 0,05%, 0,10% bzw. 0,10% betragen.

**Patentansprüche für die Vertragsstaaten: AT ES GR**

1. Verfahren zur Herstellung einer pharmazeutischen Formulierung umfassend das Vermischen von 1 bis 5 mg/ml Formulierung von 1,4-Dihydroxy-5,8-bis[2-(2-hydroxyethylamino)ethylamino]anthrachinondihydrochlorid in einer isotonischen Lösung bei einem pH von 2,0 bis 3,5, enthaltend Natriummetabisulfid mit einer Konzentration von 0,01 bis 0,10%, Dinatrium EDTA mit einer Konzentration von 0,01 bis 0,11% und Glycin mit einer Konzentration von 0,05 bis 0,20%.

2. Verfahren gemäß Anspruch 1, wobei man eine Formulierung schafft, bei der die Konzentration an 1,4-Dihydroxy-5,8-bis[2-(2-hydroxyethylamino)ethylamino]anthrachinondihydrochlorid 2 mg/ml beträgt und die Konzentrationen von Natriummetabisulfid, Dinatrium EDTA und Glycin 0,05%, 0,10% bzw. 0,10% betragen.

**Revendications pour les Etats contractants: BE CH DE FR GB IT LI NL SE**

1. Une formulation pharmaceutique comprenant de 1 à 5 mg par ml de formulation de dichlorhydrate de 1,4-dihydroxy-5,8-bis[2-(2-hydroxyéthylamino)éthylamino]anthraquinone dans une solution isotonique à un pH de 2,0 à 3,5, contenant du métabilsulfite de sodium à une concentration de 0,01 à 0,10%, du EDTA disodique à une concentration de 0,01 à 0,11% et de la glycine à une concentration de 0,05 à 0,20%.

2. Une formulation selon la revendication 1, selon laquelle la concentration du dichlorhydrate de 1,4-dihydroxy-5,8-bis[2-(2-hydroxyéthylamino)éthylamino]anthraquinone est de 2 mg/ml et les concentrations de métabisulfite de sodium, de EDTA disodique et de glycine sont de 0,05%, de 0,10% et de 0,10% respectivement.

**Revendications pour les Etats contractants: AT ES GR**

1. Une méthode de préparation d'une formulation pharmaceutique qui comprend le mélangeage de 1 à 5 mg par ml de formulation de dichlorhydrate de 1,4-dihydroxy-5,8-bis[2-(2-hydroxyéthylamino)éthylamino]anthraquinone, dans une solution isotonique à un pH de 2,0 à 3,5,contenant du métabilsulfite de sodium à une concentration de 0,01 à 0,10%, du EDTA disodique à une concentration de 0,01 à 0,11% et de la glycine à une concentration de 0,05 à 0,20%.

2. Une méthode selon la revendication 1, qui consiste à fournir une formulation dans laquelle la concentration de dichlorhydrate de 1,4-dihydroxy-5,8-bis[2-(2-hydroxyéthylamino)éthylamino]anthraquinone est de 2 mg/ml et les concentrations de métabisulfite de sodium, de EDTA disodique et de glycine sont de 0,05%, de 0,10% et de 0,10% respectivement.